# EUROPEAN PATENT SPECIFICATION

(11) **EP 3 955 870 B1**
(45) Date of publication and mention of the grant of the patent: **12.11.2025**
(21) Application number: 20790459.0
(22) Date of filing: 20.04.2020
(51) Int. Cl.: A61F 9/008

(54) **EXCIMER LASER FIBER ILLUMINATION**
EXCIMERLASER-FASERBELEUCHTUNG
ÉCLAIRAGE PAR FIBRE LASER À EXCIMÈRE

(30) Priority: 19.04.2019 US 201916389437
(43) Date of publication of application: 23.02.2022
(73) Proprietor: Elios Vision, Inc., Los Angeles, CA 90015 (US)
(72) Inventor: JUNGER, Johannes, 82205 Gilching (DE); ENDERS, Markus, 81245 München (DE)
(74) Representative: Nordmeyer, Philipp Werner
(86) International application number: PCT/US2020/028958
(87) International publication number: WO 2020/215064

(56) References cited:
- WO-A1-2019/060756
- DE-A1- 10 138 984
- US-A- 4 607 622
- US-A- 5 323 766
- US-A- 5 865 831
- US-A1- 2008 097 415
- US-A1- 2009 118 715
- US-A1- 2013 085 484
- US-A1- 2015 366 706
- US-A1- 2018 000 337
- US-A1- 2018 360 310
- DIETLEIN T S ET AL: "ERBIUM: YAG LASER TRABECULAR ABLATION (LTA) IN THE SURGICAL TREATMENT OF GLAUCOMA", LASERS IN SURGERY AND MEDICINE, WILEY- LISS, NEW YORK, US, vol. 23, no. 2, 27 May 1998 (1998-05-27), pages 104 - 110, XP000799477, ISSN: 0196-8092, DOI: 10.1002/(SICI)1096-9101(1998)23:2<104::AID-LSM8>3.0.CO;2-T

## Description

### Technical Field

The disclosure relates to medical devices, and, more particularly, to an excimer laser probe having an illumination means.

### Background

Glaucoma is a group of eye conditions which result in damage to the optic nerve and lead to vision loss. While glaucoma can occur at any age, it is more common in older adults and is one of the leading causes of blindness for people over the age of 60. A major risk factor in glaucoma is ocular hypertension, in which intraocular pressure is higher than normal. An elevated intraocular pressure can lead to atrophy of the optic nerve, subsequent visual field disturbances, and eventual blindness if left untreated.

Intraocular pressure is a function of the production of aqueous humor fluid by the ciliary processes of the eye and its drainage through a tissue called the trabecular meshwork. The trabecular meshwork is an area of tissue in the eye located around the base of the cornea and is responsible for draining the aqueous humor into a lymphatic-like vessel in the eye called Schlemm's canal, which subsequently delivers the drained aqueous humor into the bloodstream. Proper flow and drainage of the aqueous humor through the trabecular meshwork keeps the pressure inside the eye normally balanced. In open-angle glaucoma, the most common type of glaucoma, degeneration or obstruction of the trabecular meshwork can result in slowing or completely preventing the drainage of aqueous humor, causing a buildup of fluid, which increases the intraocular pressure. Under the strain of this pressure, the optic nerve fibers become damaged and may eventually die, resulting in permanent vision loss.

**If** treated early, it is possible to slow or stop the progression of glaucoma. Depending on the type of glaucoma, treatment options may include eye drops, oral medications, surgery, laser treatment, or a combination of any of these. For example, treatment of open-angle glaucoma may include surgical treatments, such as filtering surgery, in which an opening is created in the sclera of the eye and a portion of the trabecular meshwork is removed, and surgical implantation of stents or implants (i.e., drainage tubes), in which a small tube shunt is positioned within the eye to assist in fluid drainage. However, such treatments are highly invasive and may present many complications, including leaks, infections, hypotony (e.g., low eye pressure), and require post-operative, long-term monitoring to avoid late complications.

More recently, minimally invasive laser treatments have been used to treat glaucoma. **In** such treatments, the surgeon uses a laser to thermally modify and/or to puncture completely through various structures, including the trabecular meshwork and/or Schlemm's canal. For example, a laser trabeculostomy is a procedure in which a surgeon guides a working end of a laser fiber through a corneal incision of the eye and towards the trabecular meshwork and applies laser energy to destroy portions of the meshwork to create channels in the meshwork which allow aqueous humor to flow more freely into the Schlemm's canal. In current laser trabeculostomy procedures, the surgeon utilizes a gonio lens, a special contact lens prism, held over the eye, in combination with light, in order to visualize the working end of the laser fiber when positioning the laser fiber relative to the trabecular meshwork.

While a surgeon may have some view of the target site (i.e., the trabecular meshwork), the combination of the gonio lens and the current light source relied upon for illuminating the target site is inadequate. In particular, current procedures rely on an external beam of light (from a slit lamp) in an attempt to illuminate the anterior chamber angle where the cornea and the iris meet (i.e., the location of the trabecular meshwork). However, the external light source fails to provide a comprehensive view within the eye and is limiting. As such, a surgeon is unable to visually verify, with confidence, the position of the laser relative to the trabecular meshwork, the effectiveness of laser treatment to any given portion of the meshwork, as well as drainage of the aqueous humor upon laser treatment. For example, without proper visualization, a surgeon may position the laser too close or too far from the trabecular meshwork and/or position the laser at improper angles relative to the trabecular meshwork, resulting in unintended collateral tissue damage or the creation of channels that inadequate and do not provide the desired drainage. As a result, the laser treatment may be inadequate, as the desired drainage may not be achieved, and thus patients may require additional post-operative procedures to lower the intraocular pressure.

US 2013/085484 A1 discloses a system for reducing intraocular pressure in an eye, comprising: a visible light pattern generator, the visible light pattern generator being configured to project a visible light pattern onto a portion of the eye; a laser tool, the laser tool being configured to: make a perpendicular incision through the conjunctiva of the eye based on the visible light pattern; focus energy through the perpendicular incision to ablate a portion of a trabecular network of the eye, wherein said ablation creates a channel for outflow flow of fluid through a sclera venous sinus to reduce pressure within the eye.

### Summary

The invention is set out by the appended set of claims.
Systems of the invention include a laser probe for performing an intraocular procedure. The laser probe is a single use, disposable probe configured to be coupled to a laser source and transmit laser energy from the laser source to a target tissue for treatment thereof. The laser probe includes both a laser transmitting member and a light emitting member in a single component. In particular, the laser probe includes a fiber optic core comprising a delivery tip for transmitting laser energy from the laser source to the target tissue during a procedure. The laser probe further includes a light emitting member providing illumination in a field of view proximate to the delivery tip of the fiber core, thereby providing a clear field of view for a surgeon during laser treatment of the target tissue.

The laser probe of the present invention is particularly well suited for a laser trabeculostomy procedure. During such a procedure, it is critical that the surgeon has a clear field of view within the eye, particularly of the anterior chamber angle where the cornea and the iris meet so that the position of the laser relative to the trabecular meshwork can be clearly visualized. A surgeon may guide the delivery tip of the fiber optic core of the laser probe through a corneal incision of the eye and towards the trabecular meshwork. The light emitting member emits a visible light signal within the eye and proximate to the delivery tip, thereby illuminating a field of view in which the surgeon can better visualize positioning of the delivery tip and subsequent transmission of laser energy upon the trabecular meshwork. By providing a laser probe with an integrated lighting member, illumination is provided internally (i.e., within the eye), as opposed to current procedures which rely on an external light source, and thus provides a much more comprehensive view within the eye and the improved view of the target location. By providing an improved view, a surgeon is able to better position the delivery tip relative to the trabecular meshwork so as to achieve optimal photoablation and channel formation in the meshwork and/or Schlemm's canal. In particular, the orientation and positioning of the delivery tip is critical when attempting to create optimal channel formation in the tissue, particularly when attempting to achieve transverse placement of channels in the meshwork relative to Schlemm's canal, which will provide optimal drainage. Furthermore, the surgeon is able to visually verify, with more confidence, the effectiveness of the laser treatment by visualizing drainage of the aqueous humor as a result of the laser treatment.

One aspect of the present invention provides an excimer laser probe for performing an intraocular procedure. The intraocular procedure may include a laser trabeculostomy and thus the target tissue includes trabecular meshwork and/or Schlemm's canal. However, it should be noted that a laser probe consistent with the present disclosure can be used in any laser treatment of eye conditions, including, but not limited to, diabetic eye diseases, such as proliferative diabetic retinopathy or macular oedema, cases of age-related macular degeneration, retinal tears, and retinopathy of prematurity, and laser-assisted in situ keratomileusis (LASIK) to correct refractive errors, such as short-sightedness (myopia) or astigmatism.

The laser probe includes a fiber optic core comprising a proximal end couplable to an excimer laser source and a distal end comprising a delivery tip for transmitting laser energy from said excimer laser source to a target tissue for treatment thereof. The laser probe further includes an illumination member for providing illumination in a field of view proximate to said delivery tip of said fiber core.

In some embodiments, the illumination member comprises an optical fiber for receipt of a light signal from an illumination source. The illumination source provides a light signal within the visible light spectrum. Accordingly, the illumination source may include, but is not limited to, an incandescent light source, a fluorescent light source, a halogen light source, a high-intensity discharge light source, a metal halide light source, and a light emitting diode (LED) light source.

In some embodiments, the optical fiber is coaxially aligned with the fiber core. In other embodiments, the optical fiber is adjacent to the fiber core. The laser probe further includes an outer jacket surrounding the optical fiber and fiber core.

Another aspect of the present invention provides an excimer laser system for performing an intraocular procedure. Again, the intraocular procedure may include a laser trabeculostomy and thus the target tissue includes trabecular meshwork and/or Schlemm's canal. The excimer laser system includes an excimer laser source, an illumination source, and a disposable, single use probe operably couplable to the excimer laser source and illumination source and configured to be used in the intraocular procedure. The laser probe includes a fiber optic core comprising a proximal end couplable to the excimer laser source and a distal end comprising a delivery tip for transmitting laser energy from said excimer laser source to a target tissue for treatment thereof. The laser probe further includes an illumination member for receiving an illumination signal from the illumination source and for providing illumination in a field of view proximate to said delivery tip of said fiber core.

In some embodiments, the illumination member comprises an optical fiber for receipt of a light signal from an illumination source. The illumination source provides a light signal within the visible light spectrum. Accordingly, the illumination source may include, but is not limited to, an incandescent light source, a fluorescent light source, a halogen light source, a high-intensity discharge light source, a metal halide light source, and a light emitting diode (LED) light source.

In some embodiments, the optical fiber is coaxially aligned with the fiber core. In other embodiments, the optical fiber is adjacent to the fiber core. The laser probe further includes an outer jacket surrounding the optical fiber and fiber core.

### Brief Description of the Drawings

FIG. 1 is schematic sectional view of an eye illustrating the interior anatomical structure.
FIG. 2 is a perspective fragmentary view of the anatomy within the anterior chamber of an eye depicting the comeoscleral angle.
FIG. 3 diagrams an excimer laser system of the present disclosure.
FIG. 4 shows an embodiment an excimer laser system.
FIG. 5 shows an embodiment of a probe for use with the excimer laser system.
FIG. 6 shows an embodiment of a probe for use with the excimer laser system.
FIG. 7 shows a cross-sectional view of the probe taken along line A-A of FIG. 6.
FIG. 8 shows a cross-sectional view of the probe taken along line B-B of FIG. 6.
FIG. 9 shows an enlarged view of the delivery tip of a probe emitting both visible light for illuminating a field of view and laser energy for photoablation of a target tissue.

### Detailed Description

The invention provides a laser probe. The laser probe is a single use, disposable probe configured to be coupled to a laser source and transmit laser energy from the laser source to a target tissue for treatment thereof. The laser probe includes both a laser transmitting member and an illumination member in a single component. In particular, the laser probe includes a fiber optic core comprising a delivery tip for transmitting laser energy from the laser source to the target tissue during a procedure. The laser probe further includes a light emitting member providing illumination in a field of view proximate to the delivery tip of the fiber core, thereby providing a clear field of view for a surgeon during laser treatment of the target tissue.

The laser probe of the present invention is particularly well suited for intraocular procedures in which laser treatment of target tissues is desired. In particular, the laser probe of the present invention is preferably used for treating glaucoma and useful in performing a laser trabeculostomy. However, it should be noted that a laser probe consistent with the present disclosure can be used in any laser treatment of eye conditions, including, but not limited to, diabetic eye diseases, such as proliferative diabetic retinopathy or macular oedema, cases of age-related macular degeneration, retinal tears, and retinopathy of prematurity, and laser-assisted in situ keratomileusis (LASIK) to correct refractive errors, such as short-sightedness (myopia) or astigmatism.

During a laser trabeculostomy procedure, it is critical that the surgeon has a clear field of view within the eye, particularly of the anterior chamber angle where the cornea and the iris meet so that the position of the laser relative to the trabecular meshwork can be clearly visualized. By using the laser probe of the present invention, a surgeon may guide the delivery tip of the fiber optic core of the laser probe through a corneal incision of the eye and towards the trabecular meshwork. The light emitting member emits a visible light signal within the eye and proximate to the delivery tip, thereby illuminating a field of view in which the surgeon can visualize, with the aid of a gonio lens, positioning of the delivery tip and subsequent transmission of laser energy upon the trabecular meshwork. By providing a laser probe with an integrated lighting member, illumination is provided internally (i.e., within the eye), as opposed to current procedures which rely on an external light source, and thus provides a much more comprehensive view within the eye and the improved view of the target location. By providing an improved view, a surgeon is able to better position the delivery tip relative to the trabecular meshwork so as to achieve optimal photoablation and channel formation in the meshwork and/or Schlemm's canal. In particular, the orientation and positioning of the delivery tip is critical when attempting to create optimal channel formation in the tissue, particularly when attempting to achieve transverse placement of channels in the meshwork relative to Schlemm's canal, which will provide optimal drainage. Furthermore, the surgeon is able to visually verify, with more confidence, the effectiveness of the laser treatment by visualizing drainage of the aqueous humor as a result of the laser treatment.

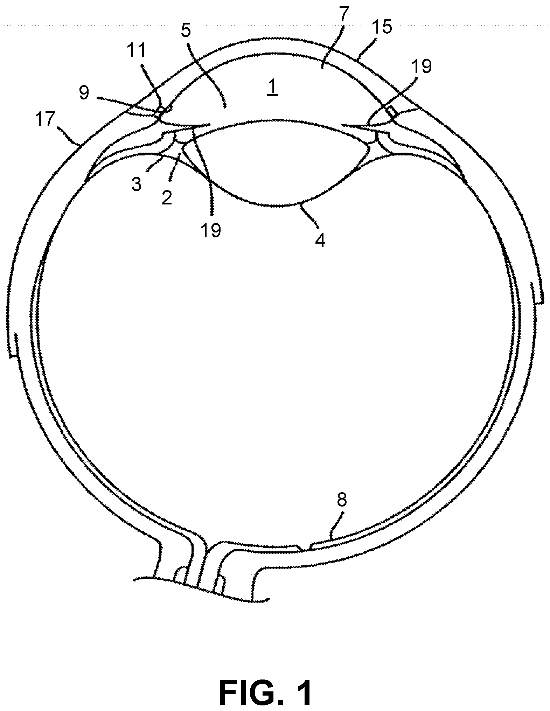

In order to fully appreciate the present invention, a brief overview of the anatomy of the eye is provided. FIG. 1 is schematic sectional view of an eye illustrating the interior anatomical structure. As shown, the outer layer of the eye includes a sclera 17 that serves as a supporting framework for the eye. The front of the sclera includes a cornea 15, a transparent tissue that enables light to enter the eye. An anterior chamber 7 is located between the cornea 15 and a crystalline lens 4. The anterior chamber 7 contains a constantly flowing clear fluid called aqueous humor 1. The crystalline lens 4 is connected to the eye by fiber zonules, which are connected to the ciliary body 3. In the anterior chamber 7, an iris 19 encircles the outer perimeter of the lens 4 and includes a pupil 5 at its center. The pupil 5 controls the amount of light passing through the lens 4. A posterior chamber 2 is located between the crystalline lens 4 and the retina 8.

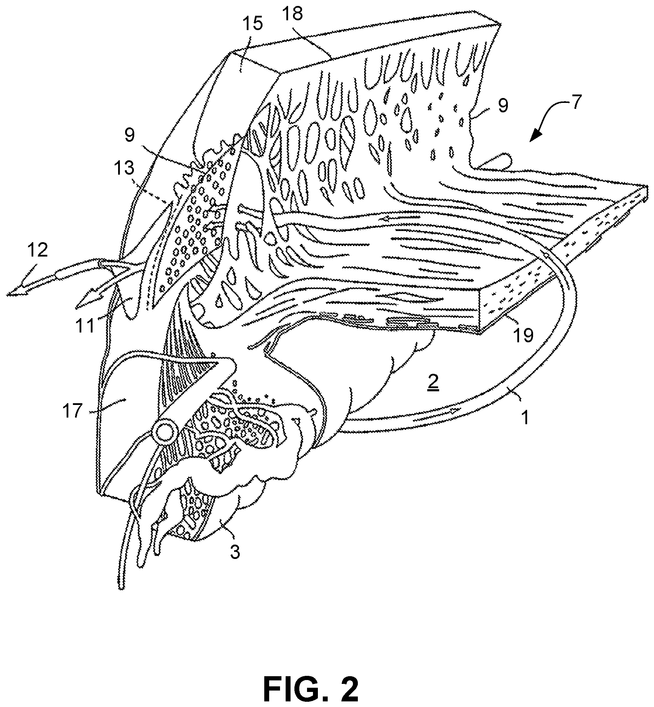

FIG. 2 is a perspective fragmentary view of the anatomy within the anterior chamber of an eye depicting the comeoscleral angle. As shown, the anatomy of the eye further includes a trabecular meshwork 9, which is a narrow band of spongy tissue that encircles the iris 19 within the eye. The trabecular meshwork has a variable shape and is microscopic in size. It is of a triangular cross-section and of varying thickness in the range of 100-200 microns. It is made up of different fibrous layers having micron-sized pores forming fluid pathways for the egress of aqueous humor. The trabecular meshwork 9 has been measured to about a thickness of about 100 microns at its anterior edge, Schwalbe's line 18, which is at the approximate juncture of the cornea 15 and sclera 17.

The trabecular meshwork widens to about 200 microns at its base where it and iris 19 attach to the scleral spur. The passageways through the pores in trabecular meshwork 9 lead through very thin, porous tissue called the juxtacanalicular trabecular meshwork 13 that in turn abuts the interior side of a structure called Schlemm's canal 11. Schlemm's canal 11 is filled with a mixture of aqueous humor and blood components and branches off into collector channels 12 which drain the aqueous humor into the venous system. Because aqueous humor is constantly produced by the eye, any obstruction in the trabecular meshwork, the juxtacanalicular trabecular meshwork or in Schlemm's canal prevents the aqueous humor from readily escaping from the anterior eye chamber which results in an elevation of intraocular pressure within the eye.

The eye has a drainage system for the draining aqueous humor 1 located in the corneoscleral angle. In general, the ciliary body 3 produces the aqueous humor 1. This aqueous humor flows from the posterior chamber 2 through the pupil 5 into the anterior chamber 7 to the trabecular meshwork 9 and into Schlemm's canal 11 to collector channels 12 to aqueous veins. The obstruction of the aqueous humor outflow which occurs in most open angle glaucoma (i.e., glaucoma characterized by gonioscopically readily visible trabecular meshwork) typically is localized to the region of the juxtacanalicular trabecular meshwork 13, which is located between the trabecular meshwork 9 and Schlemm's canal 11, more specifically, the inner wall of Schlemm's canal. It is desirable to correct this outflow obstruction by enhancing the eye's ability to use the inherent drainage system.

When an obstruction develops, for example, at the juxtacanalicular trabecular meshwork 13, intraocular pressure gradually increases over time, thereby leading to damage and atrophy of the optic nerve, subsequent visual field disturbances, and eventual blindness if left untreated. The laser probe of the present invention is well suited for use in treating glaucoma. In particular, as will be described in greater detail herein, the laser probe is configured to be coupled to a laser source and transmit laser energy from the laser source to the trabecular meshwork 13, resulting in photoablation of tissue (including at least the trabecular meshwork 13 and, in some instances, the Schlemm's canal 11) for the creation of channels in the meshwork (and potentially Schlemm's canal 11, thereby improving fluid drainage into the Schlemm's canal 11 and reducing intraocular pressure in the eye.

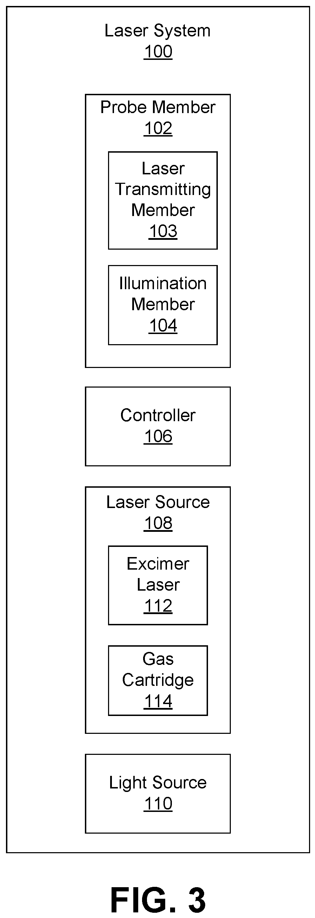

FIG. 3 diagrams an excimer laser system 100 of the present disclosure. The system 100 includes a probe member 102, which includes a laser transmitting member 103 and an illumination member 104, a controller 106, a laser source 108, and a light source 110. As will be described in greater detail herein, many of the components of the laser system 100 may be contained in a housing, such as a moveable platform, to be provided in a setting in which the procedure is to be performed (e.g., operating room, procedure room, outpatient office setting, etc.) and the probe member 102 may connect to the housing for use during treatment. Upon coupling the probe member 102 to the housing, the laser transmitting member 103 and illumination member 104 are each coupled to the respective laser source 108 and light source 110. The controller 106 provides an operator (i.e., surgeon or other medical professional) with control over the output of laser signals (from the laser source 108 to the laser transmitting member 103) and, in turn, control over the transmission of laser energy from the laser transmitting member 103 of the probe 102. The controller 106 further provides the operator with control over the output of light signals (from the light source 110 to the illumination member 104) and, in turn, control over the emission of light from the illumination member 104.

The controller 106 may include software, firmware and/or circuitry configured to perform any of the aforementioned operations. Software may be embodied as a software package, code, instructions, instruction sets and/or data recorded on non-transitory computer readable storage medium. Firmware may be embodied as code, instructions or instruction sets and/or data that are hard-coded (e.g., nonvolatile) in memory devices. "Circuitry", as used in any embodiment herein, may comprise, for example, singly or in any combination, hardwired circuitry, programmable circuitry such as computer processors comprising one or more individual instruction processing cores, state machine circuitry, and/or firmware that stores instructions executed by programmable circuitry. For example, the controller 106 may include a hardware processor coupled to non-transitory, computer-readable memory containing instructions executable by the processor to cause the controller to carry out various functions of the laser system 100 as described herein, including controller laser and/or illumination output.

The laser source 108 may include an excimer laser 112 and a gas cartridge 114 for providing the appropriate gas combination to the laser 112. The excimer laser 112 is a form of ultraviolet laser that generally operates in the UV spectral region and generates nanosecond pulses. The excimer gain medium (i.e., the medium contained within the gas cartridge 114) is generally a gas mixture containing a noble gas (e.g., argon, krypton, or xenon) and a reactive gas (e.g., fluorine or chlorine). Under the appropriate conditions of electrical stimulation and high pressure, a pseudo-molecule called an excimer (or in the case of noble gas halides, exciplex) is created, which can only exist in an energized state and can give rise to laser light in the UV range.

Laser action in an excimer molecule occurs because it has a bound (associative) excited state, but a repulsive (dissociative) ground state. Noble gases such as xenon and krypton are highly inert and do not usually form chemical compounds. However, when in an excited state (induced by electrical discharge or high-energy electron beams), they can form temporarily bound molecules with themselves (excimer) or with halogens (exciplex) such as fluorine and chlorine. The excited compound can release its excess energy by undergoing spontaneous or stimulated emission, resulting in a strongly repulsive ground state molecule which very quickly (on the order of a picosecond) dissociates back into two unbound atoms. This forms a population inversion. The excimer laser 112 of the present system 100 is an XeCl excimer laser and emits a wavelength of 308 nm.

The light source 110 provides a light signal to the illumination member 104 within the visible light spectrum. Accordingly, the illumination source 110 may include, but is not limited to, an incandescent light source, a fluorescent light source, a halogen light source, a high-intensity discharge light source, a metal halide light source, and a light emitting diode (LED) light source.

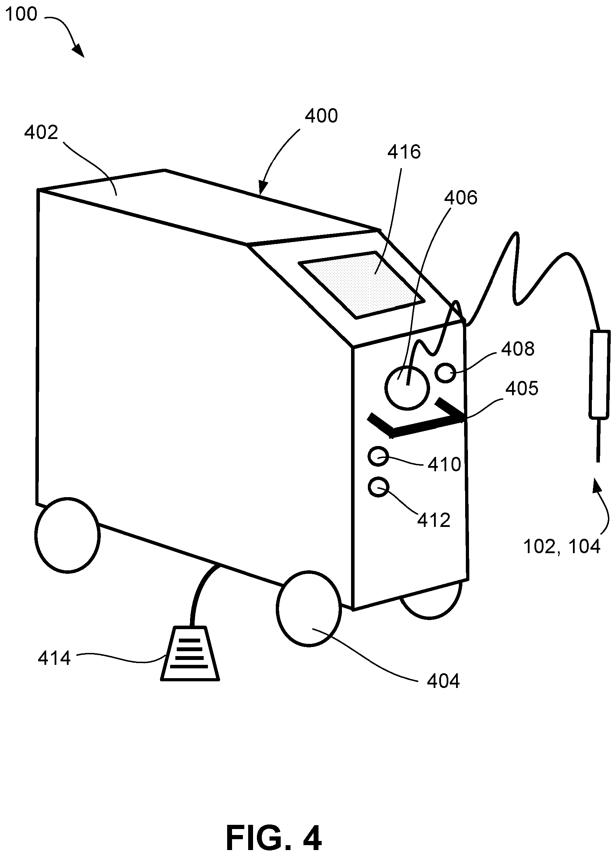

FIG. 4 shows an embodiment an excimer laser system 100 provided in an instrument 400. As previously described, one or more components of the system 100 can be contained within the instrument 400. In the present embodiment, the controller 106, the laser source 108 (including the excimer laser 112 and gas cartridge 114), and the light source 110 are contained within a housing 402. The housing 402 has wheels 404 and is portable. The instrument 400 further includes a push-pull handle 405 which assists with portability of the instrument 400. The instrument 400 further includes a connection port 406 for receiving a connecting end of the probe member 102 to establish a connection between the laser transmitting member 103 and illumination member 104 and the respective laser source 108 and light source 110. The instrument 400 further includes various inputs for the operator, such as a fiber probe cap holder 408, an emergency stop button 410, and a power switch 412. The instrument 400 further includes a foot pedal 414 extending from the housing 402 and is operable to provide control over the delivery of shots from the excimer laser 412 to the laser transmitting member 103 of the probe 102. The instrument 400 further includes a display 416, which may be in the form of an interactive user interface. In some examples, the interactive user interface 410 displays patient information, machine settings, and procedure information.

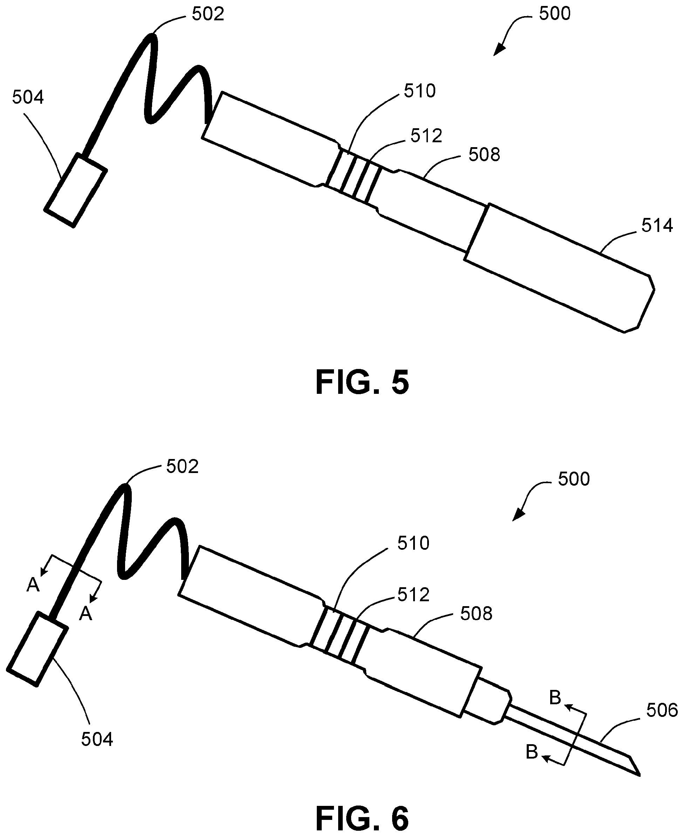

FIG. 5 shows an embodiment of a probe 500 for use with the excimer laser system 100, illustrating the probe 500 having a capped, distal delivery tip 506. FIG. 6 shows an embodiment of the probe 500 with the cap 514 removed, exposing the delivery tip 506 of the probe 500. The probe 500 is a single use, disposable unit. The probe 500 generally includes a laser transmitting member and an illumination member as previously described herein, wherein each are coupled to their respective sources (i.e., laser source 108 and light source 110) by way of a connector 502 (elongated cord) extending from the body of the probe 500 and having a connection assembly 504 configured to be received within the connection port 406 of the instrument 400. The probe 500 further includes a delivery tip 506 from which laser energy (from the laser transmitting member) and visible light (from the illumination member) may be emitted. The probe 500 includes a handheld body 508, which may include a finger grip 510 with ridges or depressions 512. The body 508 of the handheld probe 500 may be metal or plastic.

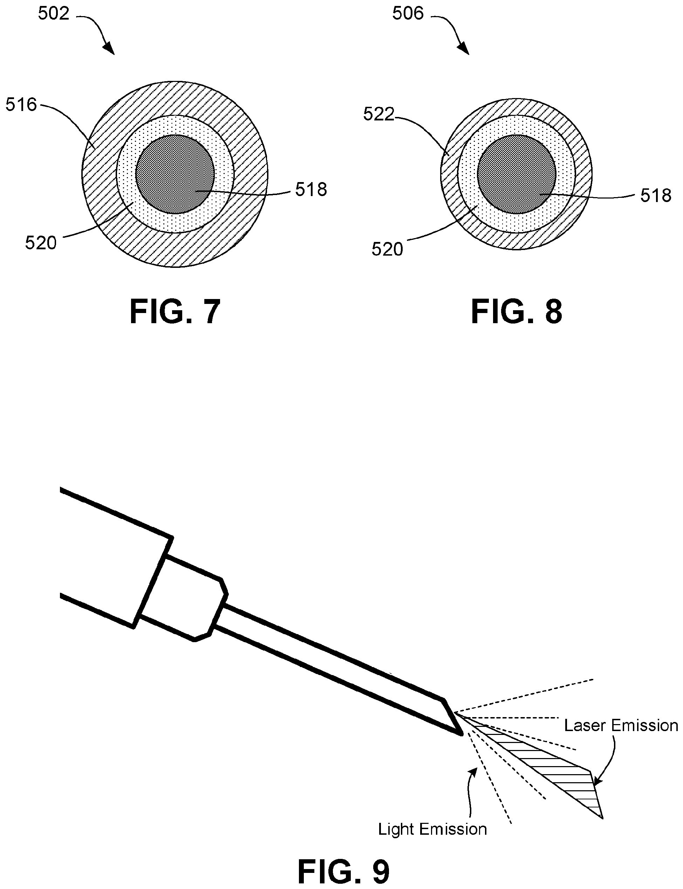

FIGS. 7 and 8 show cross-sectional views of the probe 500 taken along line A-A and line B-B of FIG. 6, respectively. As shown, the laser transmitting member may include fiber optic core 518 that runs through the fiber probe 500 and forms part of the connector 502. Similarly, the illumination member may include an optical fiber 520 that also runs through the fiber probe 500 and forms part of the connector 502. A protective sheath 516 surrounds the fiber optic core 518 and optical fiber 520. In some examples, the protective sheath 516 is a protective plastic or rubber sheath. The fiber optic core 518 and optical fiber 520 further form part of the delivery tip 506 of the probe 500. A metal jacket 522 surrounds the fiber optic core 518 and optical fiber 520. In some instances, a stainless steel jacket 522 surrounds and protects the fiber optic core 518 and optical fiber 520. As illustrated, in some embodiments, the optical fiber 520 is coaxially aligned with the fiber optic core 518, either surrounding the core 518, or, in other embodiments, the core 518 may surround the fiber 520. In other embodiments, the optical fiber 520 is adjacent to the fiber optic core 518.

FIG. 9 shows an enlarged view of the delivery tip 502 of a probe 500 emitting visible light (via emission from the optical fiber 520 upon receipt of light signals from the light source 110) and emitting laser energy (via emission from the fiber optic core 518 upon receipt of laser pulses from the laser source 108) for photoablation of a target tissue.

The laser probe of the present invention is particularly well suited for intraocular procedures in which laser treatment of target tissues is desired. In particular, the laser probe of the present invention is preferably used for treating glaucoma and useful in performing a laser trabeculostomy. However, it should be noted that a laser probe consistent with the present disclosure can be used in any laser treatment of eye conditions, including, but not limited to, diabetic eye diseases, such as proliferative diabetic retinopathy or macular oedema, cases of age-related macular degeneration, retinal tears, and retinopathy of prematurity, and laser-assisted in situ keratomileusis (LASIK) to correct refractive errors, such as short-sightedness (myopia) or astigmatism.

During a laser trabeculostomy procedure, it is critical that the surgeon has a clear field of view within the eye, particularly of the anterior chamber angle where the cornea and the iris meet so that the position of the laser relative to the trabecular meshwork can be clearly visualized. By using the laser probe of the present invention, a surgeon may guide the delivery tip of the fiber optic core of the laser probe through a corneal incision of the eye and towards the trabecular meshwork. The light emitting member emits a visible light signal within the eye and proximate to the delivery tip, thereby illuminating a field of view in which the surgeon can visualize, with the aid of a gonio lens, positioning of the delivery tip and subsequent transmission of laser energy upon the trabecular meshwork. By providing a laser probe with an integrated lighting member, illumination is provided internally (i.e., within the eye), as opposed to current procedures which rely on an external light source, and thus provides a much more comprehensive view within the eye and the improved view of the target location. By providing an improved view, a surgeon is able to better position the delivery tip relative to the trabecular meshwork so as to achieve optimal photoablation and channel formation in the meshwork and/or Schlemm's canal. **In** particular, the orientation and positioning of the delivery tip is critical when attempting to create optimal channel formation in the tissue, particularly when attempting to achieve transverse placement of channels in the meshwork relative to Schlemm's canal, which will provide optimal drainage. Furthermore, the surgeon is able to visually verify, with more confidence, the effectiveness of the laser treatment by visualizing drainage of the aqueous humor as a result of the laser treatment.

## Claims

1. An excimer laser probe (102, 500) for performing an intraocular procedure, said laser probe (102, 500) comprising: a fiber optic core (518) comprising a proximal end couplable to an excimer laser source (108) and a distal end comprising a delivery tip (506) for transmitting laser energy from said excimer laser source (108) to a target tissue for treatment thereof; and
an illumination member (104) for providing illumination in a field of view proximate to said delivery tip (506) of said fiber core (518),
wherein the excimer laser probe (102, 500) is a disposable, single use probe.

2. The laser probe (102, 500) of claim 1, wherein said illumination member (104) comprises an optical fiber (520) for receipt of a light signal from an illumination source (110), wherein said illumination source (110) preferably provides a light signal within the visible light spectrum, wherein said illumination source (110) further preferably is selected from the group consisting of incandescent, fluorescent, halogen, high-intensity discharge, metal halide, and light emitting diode (LED).

3. The laser probe (102, 500) of claim 2, wherein said optical fiber (520) is coaxially aligned with said fiber core (518), wherein the laser probe (102, 500) preferably further comprises an outer jacket (522) surrounding said optical fiber (520) and fiber core (518).

4. The laser probe (102, 500) of claim 2, wherein said optical fiber (520) is adjacent to said fiber core (518), wherein the laser probe (102, 500) preferably further comprises an outer jacket (522) surrounding said optical fiber (520) and fiber core (518).

5. The laser probe (102, 500) of claim 1, wherein said intraocular procedure is a laser trabeculostomy, wherein said target tissue preferably comprises at least one of a trabecular meshwork (9) and Schlemm's canal (11).

6. An excimer laser system (100) for performing an intraocular procedure, said laser system (100) comprising:
an excimer laser source (108);
an illumination source (110); and
a disposable, single use probe (102, 500) operably couplable to said excimer laser source (108) and illumination source (110), said probe (102, 500) comprising:
a fiber optic core (518) comprising a delivery tip (506) for transmitting laser energy from said excimer laser source (108) to a target tissue for treatment thereof; and
an illumination member (104) for receiving an illumination signal from the illumination source (110) and for providing illumination in a field of view proximate to said delivery tip (506) of said fiber core (518).

7. The excimer laser system (100) of claim 6, wherein said illumination member (104) comprises an optical fiber (520) for receipt of a light signal from an illumination source (110).

8. The excimer laser system (100) of claim 7, wherein said illumination source (110) provides a light signal within the visible light spectrum.

9. The excimer laser system (100) of claim 8, wherein said illumination source (110) is selected from the group consisting of incandescent, fluorescent, halogen, high-intensity discharge, metal halide, and light emitting diode (LED).

10. The excimer laser system (100) of claim 7, wherein said optical fiber (520) is coaxially aligned with said fiber core (518).

11. The excimer laser system (100) of claim 10, further comprising an outer jacket (522) surrounding said optical fiber (520) and fiber core (518).

12. The excimer laser system (100) of claim 7, wherein said optical fiber (520) is adjacent to said fiber core (518).

13. The excimer laser system (100) of claim 12, further comprising an outer jacket (522) surrounding said optical fiber (520) and fiber core (518).

14. The excimer laser system (100) of claim 6, wherein said intraocular procedure is a laser trabeculostomy.

15. The excimer laser system (100) of claim 14, wherein said target tissue comprises at least one of a trabecular meshwork (9) and Schlemm's canal (11).

## Patentansprüche

1. Excimer-Lasersonde (102, 500) zum Durchführen eines intraokularen Verfahrens, wobei die Lasersonde (102, 500) umfasst: einen faseroptischen Kern (518) umfassend ein proximales Ende, das mit einer Excimer-Laserquelle (108) koppelbar ist, sowie ein distales Ende umfassend eine Abgabespitze (506) zum Übertragen von Laserenergie von der Excimer-Laserquelle (108) zu einem Zielgewebe zu dessen Behandlung; und
ein Beleuchtungselement (104) zum Bereitstellen von Beleuchtung in einem Sichtfeld in der Nähe der Abgabespitze (506) des Faserkerns (518),
wobei die Excimer-Lasersonde (102, 500) eine Einwegsonde zur einmaligen Verwendung ist.

2. Lasersonde (102, 500) nach Anspruch 1, wobei das Beleuchtungselement (104) eine optische Faser (520) zum Empfangen eines Lichtsignals von einer Beleuchtungsquelle (110) umfasst, wobei die Beleuchtungsquelle (110) vorzugsweise ein Lichtsignal innerhalb des sichtbaren Lichtspektrums bereitstellt, wobei die Beleuchtungsquelle (110) ferner vorzugsweise ausgewählt ist aus der Gruppe bestehend aus Glühlampe, Fluoreszenzlampe, Halogenlampe, Hochintensitätsentladung, Metallhalogenidlampe und Leuchtdiode (LED).

3. Lasersonde (102, 500) nach Anspruch 2, wobei die optische Faser (520) koaxial mit dem Faserkern (518) ausgerichtet ist, wobei die Lasersonde (102, 500) vorzugsweise ferner einen Außenmantel (522) umfasst, der die optische Faser (520) und den Faserkern (518) umgibt.

4. Lasersonde (102, 500) nach Anspruch 2, wobei die optische Faser (520) benachbart zum Faserkern (518) angeordnet ist, wobei die Lasersonde (102, 500) vorzugsweise ferner einen Außenmantel (522) umfasst, der die optische Faser (520) und den Faserkern (518) umgibt.

5. Lasersonde (102, 500) nach Anspruch 1, wobei das intraokulare Verfahren eine Laser-Trabekulostomie ist, wobei das Zielgewebe vorzugsweise ein Trabekelwerk (9) und/oder einen Schlemm'schen Kanal (11) umfasst.

6. Excimer-Lasersystem (100) zum Durchführen eines intraokularen Verfahrens, wobei das Lasersystem (100) umfasst:
eine Excimer-Laserquelle (108);
eine Beleuchtungsquelle (110); und
eine Einwegsonde zur einmaligen Verwendung (102, 500), die betriebsfähig mit der Excimer-Laserquelle (108) und der Beleuchtungsquelle (110) koppelbar ist, wobei die Sonde (102, 500) umfasst:
einen faseroptischen Kern (518) umfassend eine Abgabespitze (506) zum Übertragen von Laserenergie von der Excimer-Laserquelle (108) zu einem Zielgewebe zu dessen Behandlung; und
ein Beleuchtungselement (104) zum Empfangen eines Beleuchtungssignals von der Beleuchtungsquelle (110) und zum Bereitstellen von Beleuchtung in einem Sichtfeld in der Nähe der Abgabespitze (506) des Faserkerns (518).

7. Excimer-Lasersystem (100) nach Anspruch 6, wobei das Beleuchtungselement (104) eine optische Faser (520) zum Empfangen eines Lichtsignals von einer Beleuchtungsquelle (110) umfasst.

8. Excimer-Lasersystem (100) nach Anspruch 7, wobei die Beleuchtungsquelle (110) ein Lichtsignal innerhalb des sichtbaren Lichtspektrums bereitstellt.

9. Excimer-Lasersystem (100) nach Anspruch 8, wobei die Beleuchtungsquelle (110) ausgewählt ist aus der Gruppe bestehend aus Glühlampe, Fluoreszenzlampe, Halogenlampe, Hochintensitätsentladung, Metallhalogenidlampe und Leuchtdiode (LED).

10. Excimer-Lasersystem (100) nach Anspruch 7, wobei die optische Faser (520) koaxial mit dem Faserkern (518) ausgerichtet ist.

11. Excimer-Lasersystem (100) nach Anspruch 10, ferner umfassend einen Außenmantel (522), der die optische Faser (520) und den Faserkern (518) umgibt.

12. Excimer-Lasersystem (100) nach Anspruch 7, wobei die optische Faser (520) benachbart zum Faserkern (518) angeordnet ist.

13. Excimer-Lasersystem (100) nach Anspruch 12, ferner umfassend einen Außenmantel (522), der die optische Faser (520) und den Faserkern (518) umgibt.

14. Excimer-Lasersystem (100) nach Anspruch 6, wobei das intraokulare Verfahren eine Laser-Trabekulostomie ist.

15. Excimer-Lasersystem (100) nach Anspruch 14, wobei das Zielgewebe ein Trabekelwerk (9) und/oder einen Schlemm'schen Kanal (11) umfasst.

## Revendications

1. Sonde laser à excimère (102, 500) pour effectuer une procédure intraoculaire, ladite sonde laser (102, 500) comprenant: un coeur de fibre optique (518) comprenant une extrémité proximale pouvant être couplée à une source laser à excimère (108) et une extrémité distale comprenant une pointe de distribution (506) pour transmettre l'énergie laser de ladite source laser à excimère (108) à un tissu cible pour le traitement de celui-ci; et
un élément d'éclairage (104) pour fournir un éclairage dans un champ de vision à proximité de ladite pointe de distribution (506) dudit coeur de fibre (518),
dans laquelle la sonde laser à excimère (102, 500) est une sonde jetable à usage unique.

2. Sonde laser (102, 500) selon la revendication 1, dans laquelle ledit élément d'éclairage (104) comprend une fibre optique (520) pour la réception d'un signal lumineux provenant d'une source d'éclairage (110), dans laquelle ladite source d'éclairage (110) fournit de préférence un signal lumineux dans le spectre de lumière visible, dans laquelle ladite source d'éclairage (110) est en outre de préférence choisie dans le groupe constitué par incandescente, fluorescente, halogène, décharge de haute intensité, halogénure métallique et diode électroluminescente (LED).

3. Sonde laser (102, 500) selon la revendication 2, dans laquelle ladite fibre optique (520) est alignée coaxialement avec ledit coeur de fibre (518), dans laquelle la sonde laser (102, 500) comprend en outre de préférence une gaine extérieure (522) entourant ladite fibre optique (520) et ledit coeur de fibre (518).

4. Sonde laser (102, 500) selon la revendication 2, dans laquelle ladite fibre optique (520) est adjacente audit coeur de fibre (518), dans laquelle la sonde laser (102, 500) comprend en outre de préférence une gaine extérieure (522) entourant ladite fibre optique (520) et ledit coeur de fibre (518).

5. Sonde laser (102, 500) selon la revendication 1, dans laquelle ladite procédure intraoculaire est une trabéculotomie laser, dans laquelle ledit tissu cible comprend de préférence au moins l'un d'un maillage trabéculaire (9) et du canal de Schlemm (11).

6. Système laser à excimère (100) pour effectuer une procédure intraoculaire, ledit système laser (100) comprenant:
une source laser à excimère (108);
une source d'éclairage (110); et
une sonde jetable à usage unique (102, 500) pouvant être couplée de manière opérationnelle à ladite source laser à excimère (108) et à ladite source d'éclairage (110), ladite sonde (102, 500) comprenant:
un coeur de fibre optique (518) comprenant une pointe de distribution (506) pour transmettre l'énergie laser de ladite source laser à excimère (108) à un tissu cible pour le traitement de celui-ci; et
un élément d'éclairage (104) pour recevoir un signal d'éclairage de la source d'éclairage (110) et pour fournir un éclairage dans un champ de vision à proximité de ladite pointe de distribution (506) dudit coeur de fibre (518).

7. Système laser à excimère (100) selon la revendication 6, dans lequel ledit élément d'éclairage (104) comprend une fibre optique (520) pour la réception d'un signal lumineux provenant d'une source d'éclairage (110).

8. Système laser à excimère (100) selon la revendication 7, dans lequel ladite source d'éclairage (110) fournit un signal lumineux dans le spectre de lumière visible.

9. Système laser à excimère (100) selon la revendication 8, dans lequel ladite source d'éclairage (110) est choisie dans le groupe constitué par incandescente, fluorescente, halogène, décharge de haute intensité, halogénure métallique et diode électroluminescente (LED).

10. Système laser à excimère (100) selon la revendication 7, dans lequel ladite fibre optique (520) est alignée coaxialement avec ledit coeur de fibre (518).

11. Système laser à excimère (100) selon la revendication 10, comprenant en outre une gaine extérieure (522) entourant ladite fibre optique (520) et ledit coeur de fibre (518).

12. Système laser à excimère (100) selon la revendication 7, dans lequel ladite fibre optique (520) est adjacente audit coeur de fibre (518).

13. Système laser à excimère (100) selon la revendication 12, comprenant en outre une gaine extérieure (522) entourant ladite fibre optique (520) et ledit coeur de fibre (518).

14. Système laser à excimère (100) selon la revendication 6, dans lequel ladite procédure intraoculaire est une trabéculotomie laser.

15. Système laser à excimère (100) selon la revendication 14, dans lequel ledit tissu cible comprend au moins l'un d'un maillage trabéculaire (9) et du canal de Schlemm (11).
